# EUROPEAN PATENT APPLICATION

(11) **EP 4 032 499 A1**
(43) Date of publication of application: **27.07.2022**
(21) Application number: 20864402.1
(22) Date of filing: 18.09.2020
(51) Int. Cl.: A61F 2/00, A61L 27/14, A61L 27/58, A61B 17/06

(54) **LIFTING THREAD WITH STRETCHABLE VARIABLE PORTION**

(30) Priority: 19.09.2019 KR 20190115333
(71) Applicant: Neo Dr. Inc., Wonju-si, Gangwon-do 26311 (KR)
(72) Inventor: KIM, Hyeon Ho, Seoul 05403 (KR)
(74) Representative: MERH-IP Matias Erny Reichl Hoffmann Patentanwälte PartG mbB
(86) International application number: PCT/KR2020/012673
(87) International publication number: WO 2021/054792

(57) **Abstract**

A suture thread for use in a lifting procedure according to the present invention is characterized by comprising: fixed portions composed of non-stretchable medical thread, wherein the medical thread is formed on both sides thereof and is fixed to the skin; and a variable portion that is positioned between the fixed portions and imparts structural elasticity, wherein the fixed portions and the variable portions are connected continuously, without a separate connecting structure, to form a single integrated medical thread.

## Description

### [Technical Field]

The present invention relates to a thread for thread-embedding therapy, and more particularly, a lifting thread with a stretchable and variable portion, wherein the variable portion is structurally stretchable and is formed at a part of a conventional suture thread that is relatively non-stretchable, so that a lifting portion is firmly secured without sagging even when using the conventional suture thread, while neither causing pain in the sutured site nor breaking the suture thread even when muscles move largely such as laughing or yawning.

### [Background Art]

Recently, with increased interest in skin beauty, thread-embedding therapy has been developed and widely used to improve sagging or wrinkles of the skin using medical threads.

The medical thread ("suture thread") inserted through thread-embedding therapy has effects of stimulating and pulling the surrounding skin tissue to strengthen muscles or improve sagging and wrinkles of the skin, and then is absorbed into the treated skin over time.

Further, the thread used in the thread-embedding therapy is usually operated through a thread inserter, and the thread inserter includes a tubular insertion needle that extends long in front and rear directions and is provided with a handle at the rear end thereof, and a thread of which one end is inserted into the insertion needle and the other end is exposed to the outside while extending rearward.

When sticking the insertion needle of the thread inserter into a human body, the thread is drawn into the human body by the insertion needle. When the insertion needle is removed therefrom while the thread reached a desired site of the human body, only the thread remains inside the body. As such, the thread remaining in the body pulls and secures the internal tissue of the body, thereby attaining effects of improving the sagging and wrinkles of the skin.

Meanwhile, in order to improve wrinkles or maintain elasticity of the skin tissue by inserting the thread into the body, the larger the diameter of the thread to be inserted, the greater the effects may be obtained. However, when the diameter of the inserting needle increases, pain becomes severe while causing side effects, therefore, the diameter of the insertion needle is limited and a thread inserted into the insertion needle may also be limited in diameter at a predetermined range.

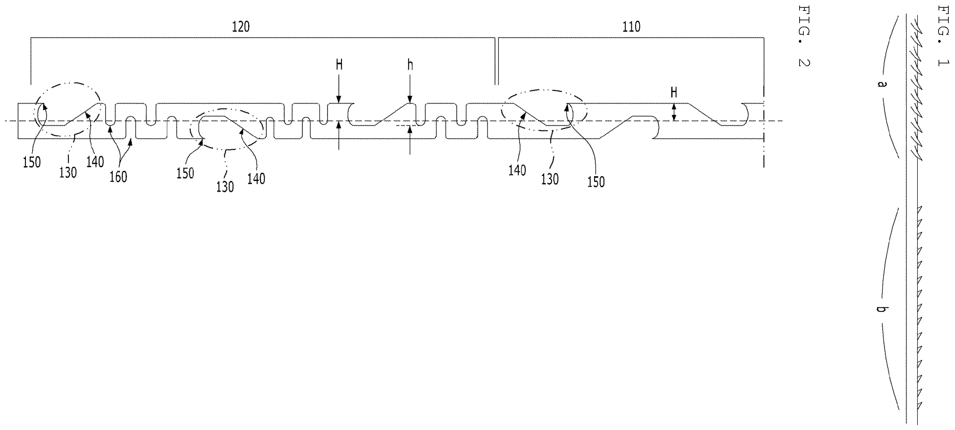

A conventional suture thread 1 for a lifting procedure in a linear shape is shown in FIG. 1. The suture thread is divided into first and second regions around a point, wherein a number of protrusions are formed and inclined at an angle facing each other around a periphery of the first region (a) and the second region (b) so that the protrusions of the first region lift the sagging skin and the protrusions of the second region serve as anchors to hold and prevent the lifted tissue from sagging again.

However, since the suture thread 1 for a lifting procedure in a linear shape as described above only has a role of pulling the tissue under the skin (that is, in a subcutaneous layer), the skin remains stiff and entails a problem that it is not possible to make a frown or smile naturally. Further, most of the suture threads are made of non-elastic materials and, if the treated muscle moves largely such as yawning or laughing loudly, pain may occur in the treated site or the tissue may be torn. Even worse, the suture thread for a lifting procedure may be broken to deteriorate lifting effects.

Accordingly, the present invention is intended to propose a new type of suture thread for a lifting procedure that can effectively solve the problem of such a conventional suture thread for a lifting procedure as described above.

### [Disclosure]

### [Technical Problem]

According to the present invention, there is provided a suture thread for a lifting procedure, including: a fixed part that has no elastic structure and can firmly lift a lifting portion without sagging; and a variable part that is structurally stretchable and is formed at a site with large movement, thereby preventing occurrence of pain and thread breakage even when the muscle (tissue) moves largely such as laughing or yawning.

Further, the present invention is intended to provide a suture thread for a lifting procedure, which can be manufactured by a simple method using pressing, molding, laser, ultrasonic processing, etc., thereby reducing the manufacturing cost.

### [Technical Solution]

The medical thread ("suture thread") for a lifting procedure according to one embodiment of the present invention may be formed to be divided into a first region and a second region in a longitudinal direction, wherein the first region includes a fixed part having a fixing groove formed with an inclined jaw at an acute angle on an outer periphery of the suture thread so that the fixed part penetrates into a subcutaneous tissue of a person to be treated ("subject") and then is fixed therein, and wherein the second region includes a variable part having structural elasticity, which has flexible grooves formed alternately in a zig-zag form on both sides of the outer periphery of the suture thread, whereby a connection portion between the flexible grooves of the variable part may be stretched when an external force is applied, thereby imparting elasticity to the suture thread.

Further, an inclined jaw at an acute angle ("acute angle jaw") that penetrates into and is fixed in the subcutaneous tissue of the subject may be further provided between the variable parts in the second region. Further, the fixing groove preferably has an asymmetric structure that includes an inclined surface and the acute angle jaw.

Further, at a position spaced apart from the acute angle jaw of the fixing groove at a predetermined interval, a concave bent groove may be formed. When the acute angle jaw penetrates into the subcutaneous tissue of the subject, the jaw may be bent and rotated in the bent groove direction to thus further protrude toward the outer periphery of the suture thread, which in turn can be firmly secured while penetrating deeper the subcutaneous tissue of the subject.

The variable part may form a spring shape by cutting out the suture thread so as to be spirally connected along the direction of the suture thread, and a depth h of the flexible groove is preferably greater than a linear distance H from the center of the suture thread to the outer periphery thereof.

### [Advantageous effects]

The suture thread for a lifting procedure according to the present invention may include a first region for firmly fixing a lifting portion without sagging using a single thread, and a second region that is structurally designed to be stretchable and is embedded in a portion with large movement, wherein the first region and the second region are integrally formed so that the lifting portion may be firmly secured to the body tissue without sagging, while preventing occurrence of pain and/or thread breakage even when the muscle (tissue) moves largely such as laughing or yawning.

Further, the present invention also has an advantage of reducing the manufacturing cost because the stretchable and variable part may be formed through pressing, molding, laser, ultrasonic processing, or the like even when using a suture thread made of a relatively inexpensive non-stretchable material.

Further, even if the suture thread of the present invention is manufactured using a thread having the same diameter, a bent groove for releasing a supporting force to support the acute angle (inclined) jaw is formed in the rear of the acute angle jaw, so that the acute angle jaw may not be pushed to the back side. Therefore, when the acute angle jaw penetrates into the body tissue, the acute angle jaw is bent and rotated in a direction of filling the bent groove to thus further protrude in the outer direction of the suture thread, which in turn may function as an anchor, thereby attaining an advantage in that the lifting portion may be more firmly secured.

### [Description of Drawings]

FIG. 1 illustrates a conventional suture thread for a lifting procedure.
FIG. 2 schematically illustrates a medical (suture) thread for a lifting procedure, which includes a first region and a second region, according to an embodiment of the present invention.
FIG. 3 is an enlarge view of the second region in which a variable part 300 and a fixed part 200 are included.
FIG. 4 is an enlarge view of the first region in which the fixed part 200 is included.
FIG. 5 is an enlarged view of the first region of the suture thread for a lifting procedure according to another embodiment of the present invention.

### [Description of reference numerals]

100: Suture thread for a lifting procedure according to the present invention.
110: First region
120: Second region
130: Fixing groove
140: Inclined surface of the fixing groove
150: Inclined jaw at acute angle of the fixing groove
160: Flexible groove
200: Fixed part
300: Variable part

### [Detailed Description of Preferred Embodiments of Invention]

Hereinafter, before describing preferred embodiments of the present invention in detail, terms or words used in the present specification and claims should not be construed as being limited to conventional or dictionary meanings, instead, should be interpreted as having the meanings and concepts consistent with the technical idea of the present invention.

Throughout the present specification, when a certain part "includes" a component, it means that other components may be further included rather than excluding the same unless specifically stated to the contrary.

Hereinafter, a preferred embodiment of the present invention will be described with reference to FIG. 2. However, the scope of the present invention is not limited to the following preferred embodiments, and those skilled in the art may implement various modified forms of the contents described in the present specification within the scope of the present invention.

As shown in FIG. 2, the suture thread for a lifting procedure, that is, a medical thread for a lifting procedure according to the present invention may include: a first region 110 in which a plurality of fixed parts 200 are repeatedly formed; and a second region 120 in which fixed parts 200 and variable parts 300 are formed alternately and repeatedly.

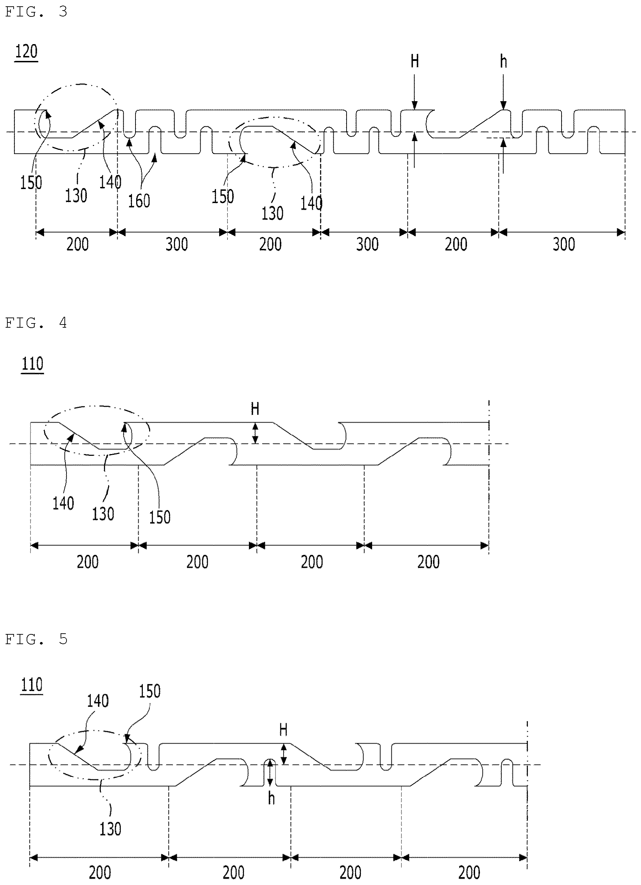

Each of the fixed parts 200 formed in the first region 110 and a part of the second region 120 may include a fixing groove 130 that is concave on an outer periphery of the suture thread, wherein the fixing groove 130 is preferably formed with an asymmetrical structure including an inclined surface 140 and an inclined jaw 150 at an acute angle ("an acute angle jaw") (see FIG. 4).

The acute angle jaw 150 formed on one side of the fixing groove 130 may be a sharp jaw inclined at an acute angle of less than 90°, so that the acute angle jaw can penetrate into a body tissue and may serve as an anchor to secure the tissue during the medical procedure.

Further, in the second region 120, a variable part including U-shaped or V-shaped flexible grooves may be formed in a zig-zag manner on both sides of the suture thread so that, when an external force is applied, a connection portion between the flexible grooves may be stretched like a spring to thus have structural elasticity. Further, the fixing groove having the acute angle jaw may be formed between the variable parts, and may serve to firmly secure the body tissue while penetrating the same at the lifting portion.

Further, it should be noted that, in some cases, the first region and the second region may be repeatedly aligned in this order in the longitudinal direction of the suture thread; otherwise, another configuration consisting of at least one or more first and second regions, for example, a first region, a second region and then another first region, may also be implemented, therefore, both of the above configurations may fall within the scope of the present invention.

Moreover, when the first region having a fixing groove is provided on both sides of the second region having the variable part, the second region may be provided only with the variable part while the fixing groove may be formed only in the first region.

The acute angle jaw 150 formed on one side of the fixing groove 200 may be prepared through ultrasonic or microfine cut processing on the outer periphery of the suture thread.

Further, the suture thread is usually made of a non-elastic material. In some cases, even if the suture thread is made of an elastic material, the first region has a fixed part without a variable structure while the second region may include a variable part having a variable structure. Both of the above configurations may be implemented in the present invention since these can achieve the purposes of the present invention such as prevention of pain and thread breakage.

FIGS. 3 to 5 illustrate a linear yarn having a circular cross-section as an example, however, a yarn having a non-circular cross-sectional shape may also be possible in order to increase a surface area and to more effectively form protrusions. Such non-circular cross-sections may be formed in any shape of oval, triangle, square, parallelepiped, trapezoid, rhomboid, pentagon, hexagon, crosses, and the like. Such a yarn as the suture thread is typically manufactured by cutting a polymer filament formed through extrusion in a die having a circular cross-section. Therefore, it is possible to alter the cross-section of the extrusion die into desired and specific cross-sectional shapes, so that a suture thread having a non-circular cross-section can also be manufactured.

The suture thread (yarn) used in the present invention may be made of a material such as polydioxanone (PDO), polylacic acid (PLA) or polyglycolide (PGA), which are biodegradable polymers.

As shown in FIG. 3, a depth h of the elastic groove 160 formed in the variable part 300 may be larger than a linear distance H from the center of the yarn used for manufacturing the suture thread for a lifting procedure to the outer periphery thereof.

In the variable part 300, as described above, a plurality of V-shaped or U-shaped bents or grooves may be formed in such a manner that the bents or grooves are alternately arranged in the left and right direction so as to alternate in the left/right or up/down directions along the longitudinal direction. Such an alternating structure may impart structural elasticity to a non-stretchable suture thread.

As shown in FIG. 3, with regard to the second region 120 of the suture thread for a lifting procedure according to the present invention, a variable part 30 provided with V-shaped or U-shaped flexible grooves alternating in the left and right directions may be present between a pair of fixed parts 200, and such basic repeating units U of the variable part 300 may be included in plural.

In another embodiment of the present invention, as shown in FIG. 5, a bent groove 160 for releasing the supporting force of the acute angle jaw may be formed at a site spaced apart from the rear of the fixing groove 130 by a predetermined distance so that, when the acute angle jaw penetrates into the body tissue, it is bent and rotated in the direction of filling the bent groove while protruding further in the outer direction, thereby penetrating deeper into the body tissue and fixing the same firmly.

The additional bent groove 160 of the fixed part 200 may be selectively applied to the suture thread for a lifting procedure according to the present invention.

In another embodiment of the present invention, the variable part 300 may be prepared by cutting out the suture thread in a spiral shape in the longitudinal direction of the suture thread 100. At this time, if the variable part 300 is processed by cutting out the suture thread 100 in a spiral shape in the longitudinal direction of the thread to form flexible grooves such that a connection portion between the flexible grooves is continued in a spring shape, structural elasticity may be effectively imparted even when using a thread made of non-elastic material.

Even when a relatively inexpensive medical thread without elasticity is used, the thread may be buried (or embedded) in a subcutaneous tissue of a subject to be treated by forming an integrated structure in which a variable part is disposed between two fixed parts, thereafter, it may have an advantage of naturally relaxing or contracting depending on movement of the muscle (tissue).

The suture thread for a lifting procedure according to the present invention is characterized in that fixed parts 200 are spaced apart from each other and formed on the outer periphery of a medical thread, and a variable part 300 having V-shape or U-shaped flexible grooves is disposed between the fixed parts, and a basic repeating unit including the above parts is repeated a predetermined number of times, so that the thread can naturally relax and contract depending on movement of the skin muscles. Further, without requiring any separate coupling structure for the suture thread, the suture thread is formed in an integrated structure along with the fixed part 200, thereby attaining unique effects of effectively contracting and relaxing the thread in a stably secured state.

Further, there are advantages of maintaining a bonding force with the skin by the fixed part 200 and, at the same time, having elastic restoration force owing to the structural elasticity of a variable part 300 that is formed continuously and integrally with the fixed part.

### [Industrial Applicability]

The present invention provides a suture thread for a lifting procedure, characterized in that it can firmly lift a lifting portion without sagging by a fixed part without any stretchable structure, while forming a variable part structurally stretchable at a site with large movement whereby it can prevent occurrence of pain and thread breakage even when the muscle (tissue) move largely such as laughing or yawning, and therefore, it is proposed a suture thread for a lifting procedure which is divided into a first region and a second region in a longitudinal direction of the thread, and has technical features in that a connection portion between flexible grooves of the variable part may be stretched when an external force is applied, thereby imparting elasticity to the suture thread. Accordingly, the present invention has industrial applicability.

## Claims

1. A suture thread for a lifting procedure, which is formed to be divided into a first region and a second region in a longitudinal direction,
wherein the first region includes a fixed part having a fixing groove formed with an inclined jaw at an acute angle ("acute angle jaw") on an outer periphery of the suture thread so that the fixed part penetrates into a subcutaneous tissue of a person to be treated ("subject") and then is fixed therein, and
wherein the second region includes a variable part having structural elasticity, which has flexible grooves formed alternately in a zig-zag form on opposite sides of the outer periphery of the suture thread,
whereby a connection portion between the flexible grooves of the variable part is stretched when an external force is applied, thereby imparting elasticity to the suture thread.

2. The suture thread according to claim 1, wherein the fixing groove having the acute angle jaw is formed between the variable parts in the second region so as to penetrate into the subcutaneous tissue of the subject and then to be secured therein.

3. The suture thread according to claim 1 or 2, wherein the fixing groove has a non-symmetric structure including an inclined jaw at an acute angle to the inclined surface.

4. The suture thread according to claim 3, wherein a concave bent groove is formed at a position spaced in the rear of the acute angle jaw of the fixing groove,
so that, when the acute angle jaw penetrates into the subcutaneous tissue of the subject, the jaw is bent and rotated to thus further protrude in the outer peripheral direction of the suture thread, which in turn is firmly secured while penetrating deeper into the subcutaneous tissue of the subject.

5. The suture thread according to claim 1, wherein the variable part has a spring shape formed by cutting out the suture thread such that the variable part is continued in a longitudinal direction of the suture thread.

6. The suture thread according to claim 1, wherein a depth h of the flexible groove is larger than a linear distance H from the center of the suture thread to the outer periphery thereof.
